# EUROPÄISCHE PATENTSCHRIFT

(11) **EP 0 546 648 B1**
(45) Veröffentlichungstag und Bekanntmachung des Hinweises auf die Patenterteilung: **16.04.1997**
(21) Anmeldenummer: 92250312.3
(22) Anmeldetag: 27.10.1992
(51) Int. Cl.: A61K 6/083, C09J 4/00

(54) **Dentalklebstoff**
Dental adhesive
Adhésif dentaire

(30) Priorität: 12.11.1991 DE 4137076
(43) Veröffentlichungstag der Anmeldung: 16.06.1993
(73) Patentinhaber: IVOCLAR AG, FL-9494 Schaan (LI)
(72) Erfinder: Salz, Ulrich, Dr., W-8995 Weissenberg (DE); Rheinberger, Volker, Dr., FL-9490 Vaduz (LI)
(74) Vertreter: UEXKÜLL & STOLBERG

(56) Entgegenhaltungen:
- EP-A- 0 244 997
- EP-A- 0 418 684
- DE-A- 3 149 797
- GB-A- 2 156 347
- US-A- 4 340 529

## Beschreibung

Die Erfindung betrifft die Verwendung einer polymerisierbaren Carbonylverbindung als Dentalklebstoff.

Bei der Behandlung von Zahnmaterial kommt es darauf an, eine dauerhafte und feste Verbindung mit dem jeweiligen Füllungsmaterial zu erreichen. Aus der Literatur sind eine Reihe von Haftsystemen und Verfahren bekannt, die eine Haftung zwischen Dentin und Composit-Füllungsmaterialien bewirken. Prinzipiell läßt sich der bekannte Stand der Technik in drei Kategorien einteilen:
1. Verfahren zur Oberflächenkonditionierung des Dentins, insbesondere zur Beseitigung bzw. zur Verfestigung der mechanisch instabilen "Smear layer" ( z.B. US-PS 4 719 149, EP 0 287 927, US-PS 4 880 660 und EP 0 348 718);
2. Verfahren zum Erreichen einer guten Haftung an Dentin durch Komplexierung der Ca²⁺-Ionen an der Dentinoberfläche, wobei häufig Phosphorverbindungen (z.B. US-PS 4 816 495, DE-PS 34 14 163, DE-OS 31 50 285, DE-PS 28 18 068 und EP 0 132 318), Carbonsäuren (z.B. US-PS 4 148 988, US-PS 3 527 737 und EP 0 325 038) oder Carbonsäurederivate (z.B. US-PS 4 521 550, EP 0 206 362, EP 0 377 072, EP 0 310 919 und EP 0 348 166) eingesetzt werden.
   In manchen Fällen werden auch Phosphorverbindungen mit Carbonsäuren kombiniert (z.B. EP 0 423 430). Die komplexierende Gruppe ist in der Regel in ein Monomer eingebaut.
3. Klebstoffe, die eine covalente Bindung zwischen dem Collagen des Dentins und dem Füllungscomposit ergeben, beispielsweise durch Verwendung von Aldehydgruppen enthaltenden Verbindungen (z.B. EP 0 141 324).
   Eine weitere Möglichkeit zur Realisierung einer dauerhaften Dentinhaftung wurde darin gesucht, zusätzlich zur Aldehydreaktion eine gezielte Pfropfung von Collagen durch mit Tri-n-butylboran initiierte Pfropfcopolymerisation von Methacrylsäureestern durchzuführen (z.B. US-PS 4 830 616). Bevorzugt sind allgemein solche Monomere, die sowohl eine gute Interpenetration als auch eine gute Bindung zum Dentin-Substrat aufweisen.
   Kombinationen aus 1. und 2. bzw. 1. und 3. sind häufig. Neuerdings werden auch Kombinationen von 2. und 3. beansprucht (z.B. US-PS 4 814 423).

Derartige Systeme und Verfahren sind in großem Umfang in-vitro und in-vivo getestet worden. Derzeit ist jedoch kein Klebstoffsystem bekannt, mit dem eine ausreichende Haftkraft und gleichzeitig eine zufriedenstellende Randdichtigkeit erzielt werden kann.

Die Verbesserungswürdigkeit der bei Anwendung der bekannten Systeme und Verfahren erzielten Haftung zeigt sich darin, daß in der Regel nur adhäsive Brucharten (Bruch an der Kontaktfläche zwischen dem Dentin und dem Composit) zu beobachten sind.

Aus der DE 31 49 797 A1 ist eine Zusammensetzung bekannt, die Acetacetoxyalkyl(meth)acrylate umfaßt und zusätzlich in Reaktivklebstoffen übliche Polymere und ggf. übliche andere reaktive Monomerkomponenten enthält. Die Polymere sind Poly(meth)acrylate, (Meth)acrylatcopolymere, Polychloropren, chlorsulfoniertes Polyethylen, Nitrilkautschuke und Urethane. Als weitere Bestandteile sind reaktive Monomerkomponenten Verbindungen genannt.

Aus der EP-A-0 244 997 ist ein Haftkleber bekannt, der ein Polymer umfaßt, das als Monomer beispielsweise Actoacetoxyethylmethacrylat (AAEMA) und zusatzlich weitere polymerisierte, olefinisch ungesättigte Mono- und/oder Polycarbonsäureester enthält. Das Polymer wird als Haftklebstoff eingesetzt.

Der Erfindung liegt die Aufgabe zugrunde, einen Dentalklebstoff zur Verfügung zu stellen, bei dessen Verwendung im Vergleich zu den bekannten Dentalklebstoffen eine wesentlich höhere Haftung des eingesetzten Dentalwerkstoffs am Dentin oder Zahnschmelz und eine bessere Randdichtigkeit erzielt werden kann.

Diese Aufgabe wird gelöst, indem eine polymerisierbare mono- oder polyfunktionelle Carbonylverbindung, die
a) mindestens eine polymerisierbare Vinylgruppe und
b) mindestens eine Carbonylgruppe und in β-Position dazu eine zweite Carbonylgruppe oder eine andere funktionelle Gruppe (d.h. eine β-Carbonylstruktur) aufweist, wobei die Carbonylverbindung einer der folgenden allgemeinen Formeln enspricht: 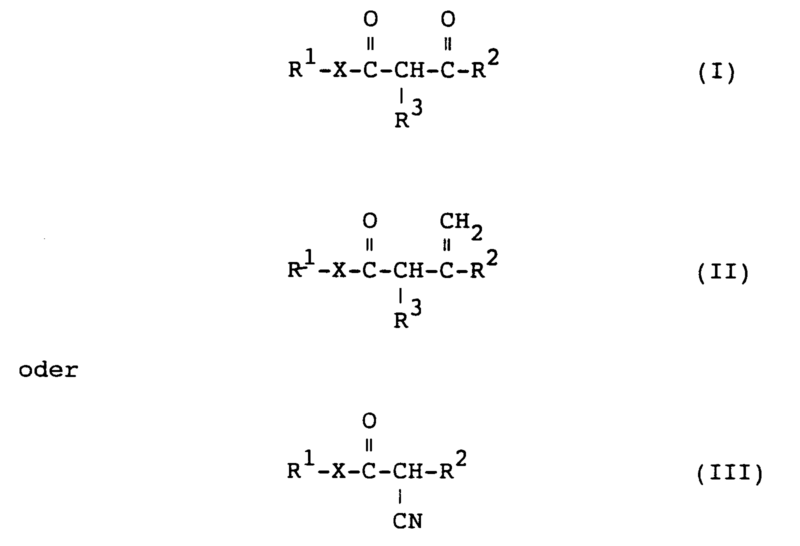 in denen
   - R¹: eine Alkyl-, Cycloalkyl-, Alkoxy-, Arylgruppe oder eine Kombination daraus sein kann,
   - R²: eine Alkyl-, Cycloalkyl-, Alkoxy- oder Arylgruppe, eine Kombination daraus oder Wasserstoff sein kann,
   - R³: eine Alkyl-, Cycloalkyl-, Alkoxy- oder Arylgruppe, eine Kombination daraus oder Wasserstoff sein kann, und
   - X: entweder nicht vorhanden ist oder O, NR, NH oder S sein kann, wobei R eine Alkyl-, Cycloalkyl-, Alkoxy- oder Arylgruppe oder eine Kombination daraus sein kann und
   wobei mindestens einer der Reste R¹, R² oder R³ außerdem durch einen mindestens eine polymerisierbare Vinylgruppe enthaltenden Rest Y substituiert ist,
ausgenommen Verbindungen, die mit Carbonsäureanhydridfunktionen substituierte Naphthylgruppen enthalten, zum dauerhaften und festen Verbinden eines zu behandelden Zahnmaterials mit einem Dentalwerkstoff verwendet wird.

Die erfindungsgemäß verwendbaren Carbonylverbindungen lassen sich in mehrere Klassen unterteilen:
a) monofunktionelle Verbindungen, d.h. monofunktionell hinsichtlich der polymerisierbaren Vinylgruppe und der β-Carbonylstruktur;
b) polyfunktionelle Verbindungen, welche
   α) monofunktionell hinsichtlich der polymerisierbaren Vinylgruppe und polyfunktionell hinsichtlich der β-Carbonylstruktur
   β) polyfunktionell hinsichtlich der polymerisierbaren Vinylgruppe und monofunktionell hinsichtlich der β-Carbonylstruktur
   γ) polyfunktionell hinsichtlich der polymerisierbaren Vinylgruppe und polyfunktionell hinsichtlich der β-Carbonylstruktur sind.

Die Alkylgruppe, die verzweigt oder unverzweigt sein kann, ist vorzugsweise eine C₁-C₂₀-, insbesondere eine C₁-C₁₀-, am meisten bevorzugt eine C₁-C₆-Alkylgruppe und die Cycloalkylgruppe ist vorzugsweise eine C₅-C₂₀-, insbesondere eine C₅-C₁₀- und besonders bevorzugt eine C₅-C₆-Cycloalkylgruppe. Die Alkoxygruppe, deren Alkylgruppe verzweigt oder unverzweigt sein kann, ist vorzugsweise eine C₁-C₂₀-, insbesondere eine C₁-C₁₀-, besonders bevorzugt eine C₁-C₆-Alkoxygruppe und die Arylgruppe ist vorzugsweise eine C₅-C₃₀-, insbesondere eine C₅-C₁₅- Arylgruppe und besonders bevorzugt eine Phenylgruppe. Auch heterocyclische Alkyl- und Arylgruppen sind geeignet.

Beispielsweise ist Y ein unsubstituierter oder substituierter Acryl- oder Methacrylsäure-, Styryl-, Vinyl- oder Allylrest. Als Substituenten kommen Carboxylgruppen, die Teil einer Ester-, Amid- oder Thioestergruppe sein können, Hydroxygruppen, Halogenatome, Alkylgruppen, insbesondere C₁-C₁₀- und bevorzugt C₁-C₆-Alkylgruppen, und/oder Cyanidgruppen in Frage.

In bevorzugten Ausführungsformen ist Y ein Acryl- oder Methacrylsäurerest, wobei die Verknüpfung mit den Substituenten R¹, R² und/oder R³ über die Carboxylgruppe erfolgt.

Die monofunktionellen Verbindungen a) haben beispielsweise die allgemeine Formel: 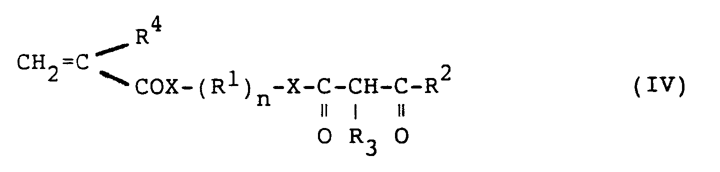
- R¹ =: Alkylen, Cycloalkylen, Alkylenoxy, Arylen,
- R² =: Alkyl, Alkoxy, Aryl, H,
- R³ =: Alkyl, Alkoxy, Aryl, H,
- R⁴ =: Alkyl, Hal, H, CN,
- X =: keine Bedeutung oder O, NR, NH, S, wobei R wie in Anspruch 1 definiert ist,
- n =: 1 und im Fall einer Alkylenoxygruppe bis zu 50,
wobei jedes X gleich oder verschieden sein kann, n vorzugsweise gleich 1 bis 10 ist und R⁴ eine Alkylgruppe der oben genannten Art, vorzugsweise Methyl oder Ethyl ist.

Zu dieser Verbindungsklasse der β-Dicarbonylacrylsäurederivate gehören u.a. die folgenden Verbindungen:

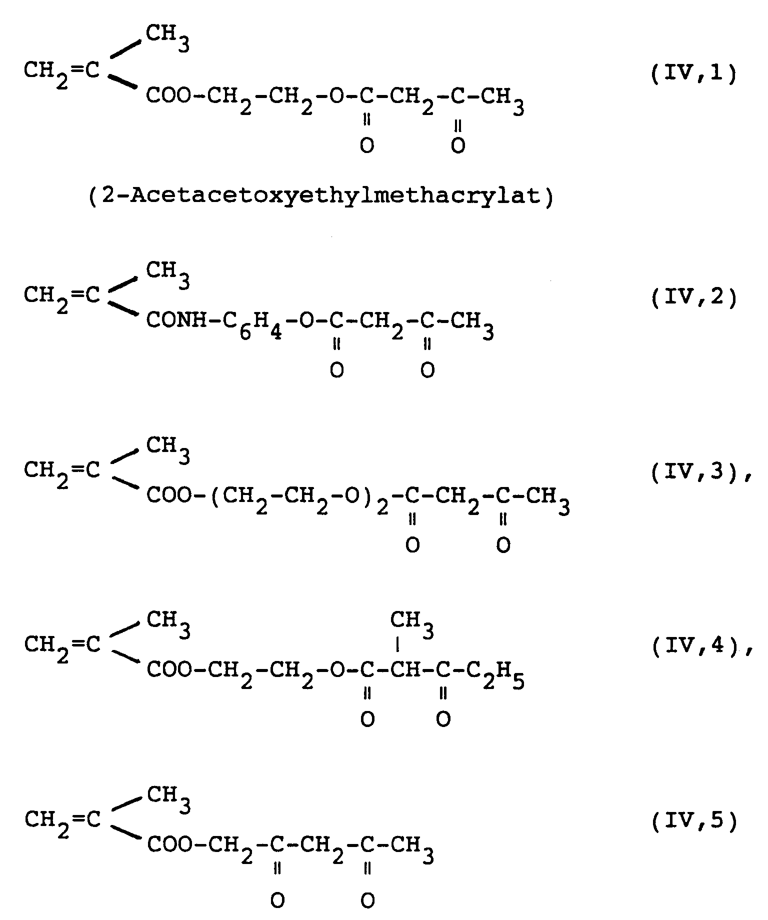

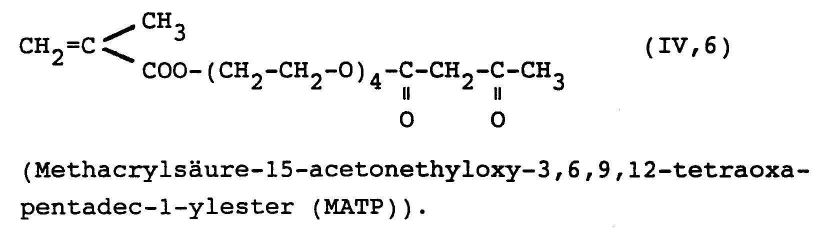

Von diesen Verbindungen sind insbesondere das 2-Acetacetoxyethylmethacrylat (IV,1) und der Methacrylsäure-15-acetonethyloxy-3,6,9,12-tetraoxapentadec-1-ylester (MATP) bevorzugt.

Weitere geeignete Acrylsäurederivate weisen die folgende allgemeine Formel auf: 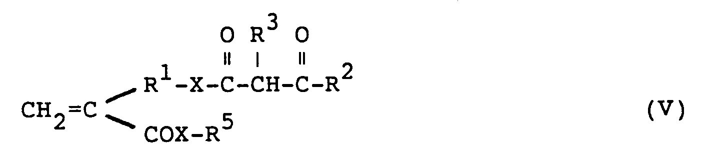 mit
- R¹ =: Alkylen, Alkenoxy, Arylen,
- R² =: Alkyl, Alkoxy, Aryl, H,
- R³ =: Alkyl, Alkoxy, Aryl, H,
- R⁵ =: Alkyl, Cycloalkyl, Alkoxy, Aryl,
- X =: O, NR, NH, S oder nicht vorhanden ist (R wie oben definiert).

Ein Beispiel hierfür ist die Verbindung der Formel:

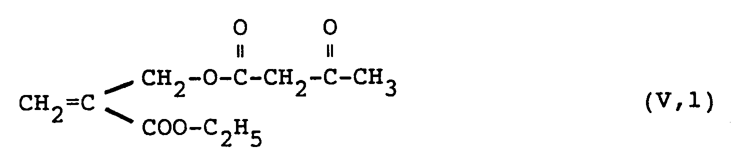

Auch Verbindungen, bei denen der Acrylsäurerest über den Rest R² oder R³ an den die β-Carbonylstruktur tragenden Molekülteil gebunden sind, können eingesetzt werden. Diese Verbindungen haben die allgemeinen Formeln: 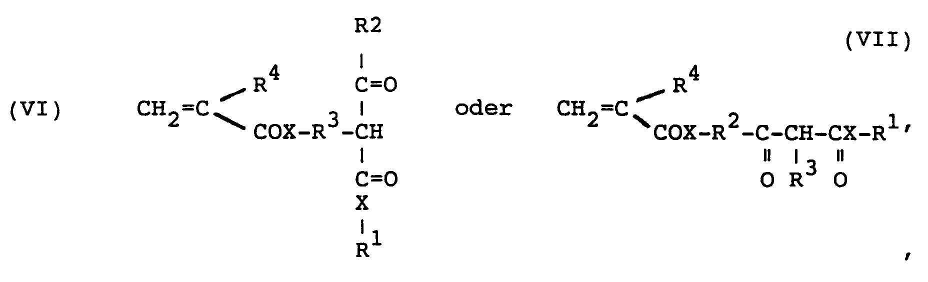 in denen die Substituenten R¹, R², R³, R⁴ und X die oben genannten Bedeutungen haben können.

Ferner gehören zu den erfindungsgemäß verwendbaren Carbonylverbindungen solche, bei denen Y anstelle eines Acrylsäurerestes ein Styryl-, Vinyl- und Allylrest ist. Diese Verbindungen haben eine der folgenden allgemeinen Formeln: 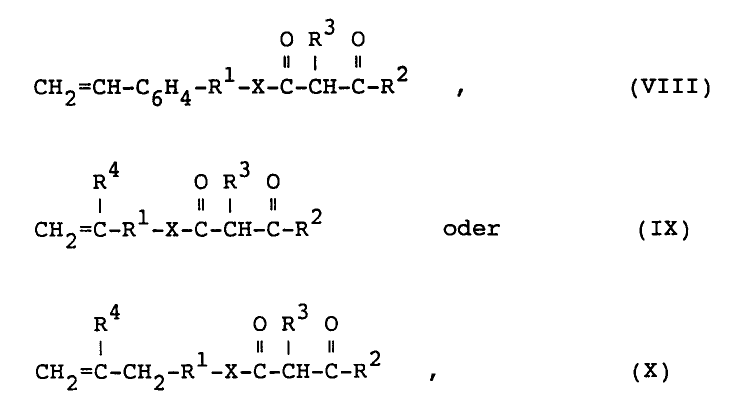 in denen R¹, R², R³, R⁴ und X die genannten Bedeutungen haben können und R¹ ohne Bedeutung sein kann.

Auch hier können die Styryl-, Vinyl- und Allylreste alternativ über einen der Reste R² oder R³ gebunden sein.

Beispiele hierfür sind:

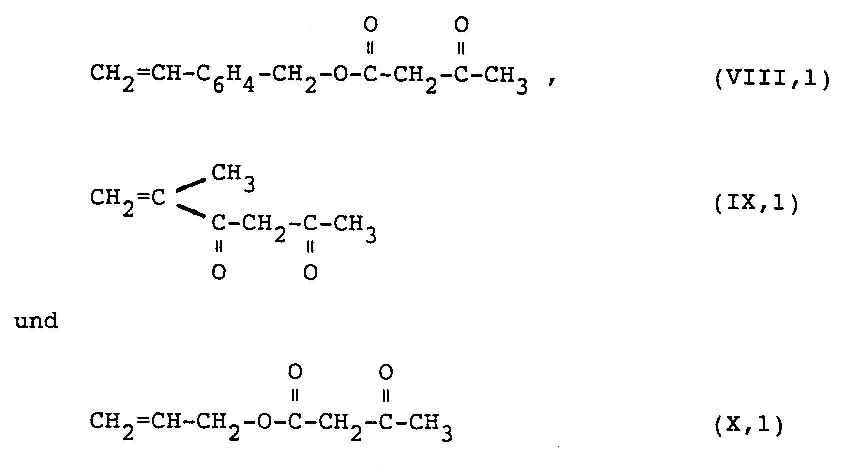

Außer den bereits genannten in der β-Carbonyl- und der Doppelbindungsfunktion monofunktionellen Carbonylverbindungen sind auch polyfunktionelle β-Carbonylverbindungen b) geeignet. Diese können sowohl hinsichtlich der β-Carbonylstruktur als auch hinsichtlich der polymerisierbaren Vinylgruppe polyfunktionell sein.
- α) -: monofunktionell hinsichtlich der polymerisierbaren Vinylgruppe und polyfunktionell hinsichtlich der β-Carbonylstruktur:

Hierzu gehören beispielsweise Verbindungen der allgemeinen Formeln 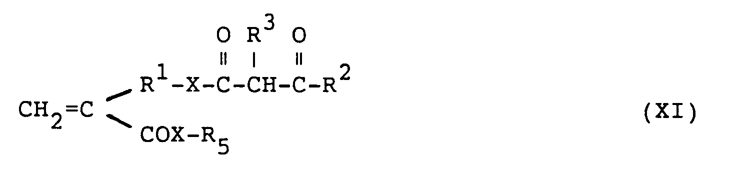 in der R⁵ = 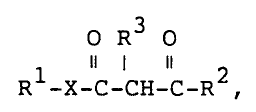 und R¹, R², R³ und X die oben genannten Bedeutungen haben, 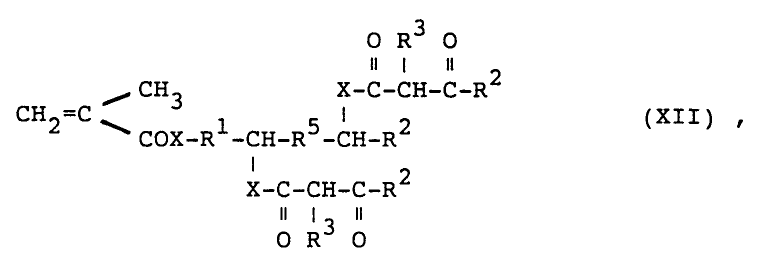 in der R¹, R², R³ und X die oben genannten Bedeutungen haben und R⁵ eine Alkylen, Cycloalkylen oder Arylengruppe der oben genannten Art ist.

Beispiele für diese Verbindungen sind: 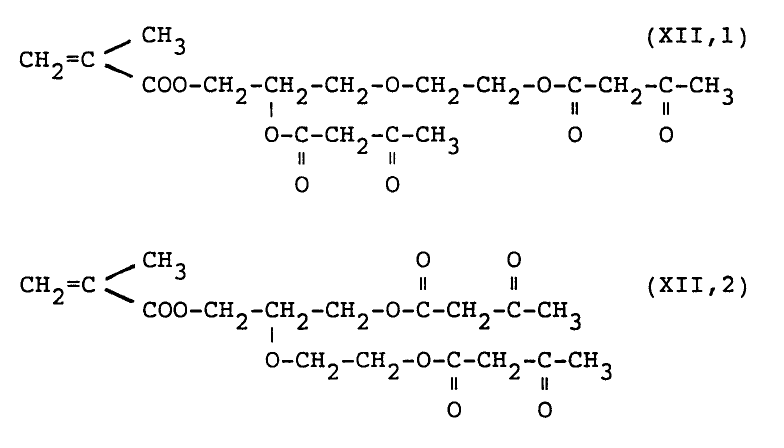
- β): - polyfunktionell hinsichtlich der polymerisierbaren Vinylgruppe und monofunktionell hinsichtlich der β-Carbonylstruktur:
Hierzu gehören beispielsweise die Verbindungen mit der allgemeinen Formel 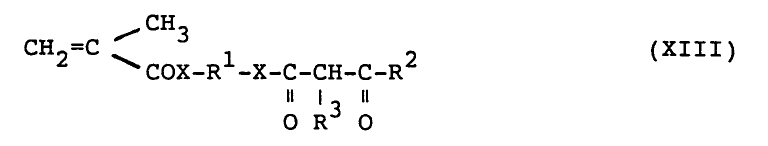 in der R¹, R², R³ und X die oben genannten Bedeutungen haben und R¹, R² und/oder R³ zusätzliche polymerisierbare Doppelbindungen enthalten, z.B. in Form von Acryl- oder Methacrylsäure-, Styryl-, Vinyl- oder Allylresten.
- γ: -: polyfunktionell hinsichtlich der polymerisierbaren Vinylgruppe und polyfunktionell hinsichtlich der β-Carbonylstruktur:
Diese können die allgemeine Formel 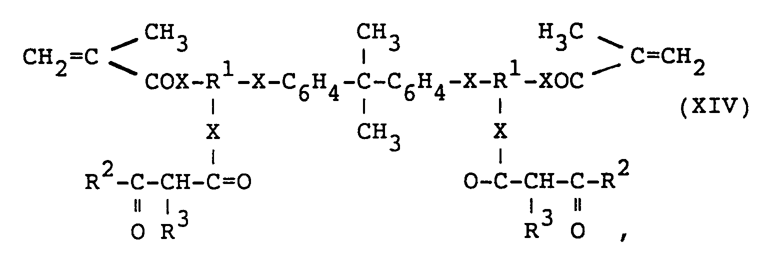 aufweisen, in der R¹ bis R³ und X die genannten Bedeutungen haben können.

Ein Beispiel hierfür ist:

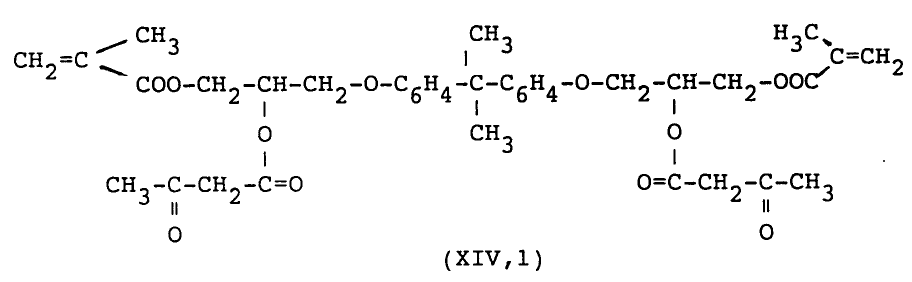

Das gängigste Verfahren zur Herstellung von β-Carbonylverbindungen der allgemeinen Formel 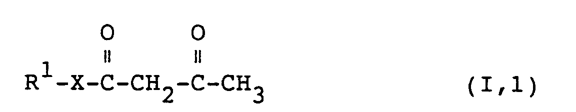 mit X = O, NH, NR und S
ist die Umsetzung einer entsprechenden HX-Verbindung mit Diketen (R.J. Clemens, Chemical Review 86, 241 (1986)).

Für β-Ketoester ist eine allgemein verwendbare Herstellungsvorschrift in der DE-OS 21 56 446 beschrieben, die gegebenenfalls leicht z.B. folgendermaßen modifiziert werden kann.

Beispielsweise erhält man die Verbindungen (IV,1) und (IV,3) in nahezu quantitativer Ausbeute, indem man 1 Mol der entsprechenden Hydroxyverbindung, 1 g Triethylamin und 1 g 2,6-Di-tert.butyl-p-kresol in 500 ml absolutem Ethylacetat löst, zu dieser Lösung innerhalb einer Stunde unter Rühren 88 g (1,05 Mol) Diketen tropft oder das entsprechend dem gewünschten Produkt modifizierte Diketen zugibt, das Reaktionsgemisch anschließend ca. 2 Stunden lang unter Rückfluß erhitzt, nach dem Abkühlen zuerst mit verdünnter HCl und anschließend mit Wasser wäscht und nach dem Trocknen mit Natriumsulfat das Lösungsmittel abdestilliert (siehe z.B. auch DE-OS 31 49 797). MATP (IV,6) ist beispielsweise durch entsprechende Umsetzung von Diketen mit Polyethylenglykol-200-monomethacrylat erhältlich.

Analog können beispielsweise p-Hydroxymethacrylsäureanilid mit Diketen oder Hydroxyethylmethacrylat (HEMA) mit 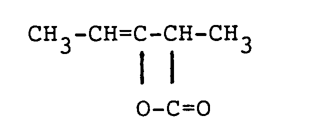 unter Erhalt von Verbindung (IV,2) oder (IV,4) und Pentan-1-ol-dion-2,4 mit Methacrylsäurechlorid oder -anhydrid unter Erhalt von Verbindung (IV,5) umgesetzt werden.

Die Darstellung von Verbindungen wie (V,1) wurde von T. Sato, N. Morita, H. Tanaka und T.Ota in Makromol. Chem. 191, 2599 (1990) beschrieben und Verbindungen wie (IX,1) lassen sich nach einer Vorschrift von A.R. Despic und Dj. Kosanovic, Makromol. Chem. 29, 151 (1959) synthetisieren.

Verbindungen wie (XII,1) und (XII,2) sind beispielsweise durch entsprechende Umsetzung von Glycidylmethacrylat (GMA) mit Ethylenglykol und anschließender Umsetzung mit Diketen und Verbindungen wie (XIV,1) durch Umsetzung von Bis-GMA mit Diketen erhältlich.

Bei Verwendung einer der oben beschriebenen Carbonylverbindungen mit mindestens einer polymerisierbaren Vinylgruppe und mindestens einer β-Carbonylstruktur, d.h. einer Carbonylgruppe und in β-Position dazu einer zweiten Carbonylgruppe oder einer funktionellen Gruppe in einem Dentalklebstoff wird im Vergleich zur Verwendung der bekannten Dentalklebstoffen eine wesentlich größere Haftung des jeweiligen Dentalwerkstoffs am zu behandelnden Zahnmaterial wie Dentin oder Zahnschmelz und eine größere Randdichtigkeit erzielt. Die erreichten Scherhaftwerte sind im Vergleich drei bis fünf mal so groß. Häufig ist bereits eine kohäsive Bruchart (Bruch im Dentin) zu beobachten. Als Dentalwerkstoffe kommen neben den bevorzugten Füllungsmaterialien beispielsweise auch Formteile wie Kronen oder Brücken in Betracht. Die Dentalwerkstoffe können auch metallisch sein.

Der verwendete Dentalklebstoff enthält etwa 1 bis 70, insbesondere 1 bis 50 und bevorzugt 5 bis 40 Gew.% der erfindungsgemäßen β-Carbonylverbindungen.

Die übrigen Bestandteile des erfindungsgemäßen Dentalklebstoffs sind üblicherweise in derartigen Haftsystemen verwendete Komponenten. Hierzu gehören z.B. PEG-200-DMA*, PEG-400-DMA*, PEG-600-DMA, PEG-1000-DMA, PEG-4000-DMA, PEG-200-Monomethacrylat, PEG-400-Monomethacrylat, Bis-GMA, Polypropylenglykol-1000-DMA, Polypropylenglykol-1000-Monomethacrylat, Glycerindimethacrylat, Glycerinmonomethacrylat, HEMA, TEG-DMA, Alkohol, Aceton, Glutaraldehyd, Campherchinon etc. (PEG = Polyethylenglykol, DMA = Dimethacrylat, GMA = Glycidylmethacrylat, HEMA = Hydroxyethylmethacrylat und TEG = Tetraethylenglykol).

Kombinationen mit bereits bekannten Haftvermittlern wie beispielsweise auf den Seiten 1 und 2 unter Punkt 1, 2 und 3 beschriebenen sind ebenfalls möglich.

Bezüglich des Wirkmechanismus wird angenommen, daß die β-Carbonylfunktion mit dem Protein reagiert, also eine wesentlich höhere Reaktivität als z.B. Glutaraldehyd aufweist. Weiterhin könnten im Falle der β-Ketoester und β-Diketone die guten Komplexierungseigenschaften eine Rolle spielen. Ferner könnte die starke Aktivierung der α-Methylengruppe eine Rolle spielen.

Es hat sich als vorteilhaft erwiesen, daß Dentin vor dem Aufbringen des erfindungsgemäßen Klebstoffs mit einem Primer zu behandeln. Als geeigneter Primer hat sich beispielsweise Syntac®-Primer erwiesen.

In den folgenden Beispielen 1 bis 6 werden erfindungsgemäße Dentalklebstoff-Formulierungen beschrieben, die 2-Acetacetoxyethylmethacrylat (1 bis 3) bzw. MATP enthalten. Die Beispiele 7 und 8 sind Vergleichsbeispiele und demonstrieren die Überlegenheit der erfindungsgemäßen gegenüber bekannten Klebstoffformulierungen.

Die verwendeten Dentalklebstoff-Formulierungen wurden hergestellt, indem die einzelnen Bestandteile unter Rühren zu dem vorgelegten Lösungsmittelgemisch gegeben wurden. Danach wurde solang gerührt, bis eine homogene, klare Lösung erhalten wurde.

Für die jeweiligen Formulierungen sind die ermittelten Scherhaftwerte angegeben.

Zur Ermittlung der Scherhaftwerte wurde zuerst auf plangeschliffenen (1000er Schleifpapier) und mit einem Luftbläser getrockneten Dentinoberflächen von extrahierten, eingebetteten Zähnen der Primer in einer dünnen Schicht aufgetragen. Nach 20 Sekunden Einwirkzeit wurde kurz mit einem Luftbläser getrocknet und dann der Klebstoff wiederum in einer dünnen Schicht aufgetragen und dünn verblasen. Nach dem Auftragen und Aushärten eines lichthärtenden Bondings (Heliobond® von Vivadent) wurde eine spezielle, teilbare Teflonform (d = 4 mm, h = 6 mm) mit einer speziellen Halterung auf der Dentinoberfläche fixiert (Halterung vergleichbar mit der von D.H. Retief, J.D. Gross, E.L. Bradley und F.R. Denys in Dental Materials 2, 72 (1986) beschriebenen). Anschließend wurde ein lichthärtendes Füllungscomposit (Heliomolar, Tetras) in einem definierten Volumen und einer definierten Haftfläche schichtweise auf die Dentinoberfläche aufpolymerisiert.

Die Scherhaftwerte wurden entweder direkt nach dem Herstellen der Prüfkörper (1 Minute und 5 Minuten) oder nach entsprechend langer Lagerung in destilliertem Wasser bei 37°C bestimmt.

Bei der erfindungsgemäßen Verwendung als Dentalklebstoffe wurden bereits unmittelbar nach dem Aufpolymerisieren des Füllungscomposits sehr hohe Haftwerte erreicht. Dies ist für die klinische Anwendung von großer Bedeutung, da eine sehr hohe Initialhaftung dem Polymerisationsschrumpf des Füllungsmaterials entgegenwirkt und somit eine hohe Randdichtigkeit der Füllung erzielt werden kann. Handelsübliche Dentinklebstoffe wie Scotchbond® und Gluma® zeigen erst nach 24 Stunden Wasserlagerung eine deutlich meßbare Dentinhaftung (siehe Beispiele 7 und 8).

Es konnte aber auch gezeigt werden, daß mit dem erfindungsgemäßen System ein dauerhafter kohäsiver Verbund zu erzielen ist.

Die Bestimmung der Haftwerte erfolgte unter Verwendung einer speziellen Abschervorrichtung wie sie z.B. vergleichbar von K. Ludwig, Dental Labor 37,(5), 757 (1989) beschrieben wurde (Materialprüfgerät Zwick 1455/ Vorschubgeschwindigkeit 0,5 mm/Min.).

### Syntac®-Primer

| | |
|---|---|
| Maleinsäure | 4 Gew.% |
| TEG-DMA* | 25 Gew.% |
| Wasser | 30 Gew.% |
| Aceton | 41 Gew.% |

| | |
|---|---|
| * TEG-DMA = Tetraethylenglykol-Dimethacrylat | |

### Beispiel 1 (Kombination von 2-Acetacetoxyethylmethacrylat mit Polyethylenglykol-Dimethacrylat (PEG-DMA))

1.
a)

| | |
|---|---|
| 2-Acetacetoxyethylmethacrylat | 5 Gew.% |
| PEG-1000-DMA | 30 Gew.% |
| Ethanol/Wasser (1:1) | 65 Gew.% |
| Dentinhaftung (24h/H₂O/37°C): 21 ± 5 MPa (bei 2 von 6 Proben kohäsive Brüche) Dentinhaftung (6 Monate/H₂O/37°C): 19 ± 4 MPa (bei 3 von 6 Proben kohäsive Brüche) Dentinhaftung (5 Minuten): 11 ± 3 MPa | |

b)

| | |
|---|---|
| 2-Acetacetoxyethylmethacrylat | 10 Gew.% |
| PEG-1000-DMA | 30 Gew.% |
| Ethanol/Wasser (1:1) | 60 Gew.% |
| Dentinhaftung (24h/H₂O/37°C): 18 ± 6 MPa (bei 4 von 6 Proben kohäsive Brüche) Dentinhaftung (6 Monate/H₂O/37°C): 17 ± 5 MPa (bei 4 von 6 Proben kohäsive Brüche) | |

### Beispiel 2 (Kombination von 2-Acetacetoxyethylmethacrylat mit Glutaraldehyd)

2.
a)

| | |
|---|---|
| 2-Acetacetoxyethylmethacrylat | 10 Gew.% |
| Glutaraldehyd (50%-ig) | 40 Gew.% |
| Ethanol/Wasser (2:1) | 50 Gew.% |
| Dentinhaftung (24h/H₂O/37°C): 17 ± 4 MPa (bei 4 von 6 Proben kohäsive Brüche) Dentinhaftung (6 Monate/H₂O/37°C): 18 ± 5 MPa (bei 5 von 6 Proben kohäsive Brüche) Dentinhaftung (5 Minuten): 12 ± 2 MPa Dentinhaftung (1 Minute): 9 ± 3 MPa | |

b)

| | |
|---|---|
| 2-Acetacetoxyethylmethacrylat | 10 Gew.% |
| Glutaraldehyd (50%-ig) | 60 Gew.% |
| Ethanol/Wasser (2:1) | 30 Gew.% |
| Dentinhaftung (24h/H₂O/37°C): 16 ± 5 MPa (bei 3 von 6 Proben kohäsive Brüche) | |

c)

| | |
|---|---|
| 2-Acetacetoxyethylmethacrylat | 5 Gew.% |
| Glutaraldehyd (50%-ig) | 60 Gew.% |
| Ethanol/Wasser (2:1) | 35 Gew.% |
| Dentinhaftung (24h/H₂O/37°C): 18 ± 6 MPa (bei 3 von 6 Proben kohäsive Brüche) | |

### Beispiel 3 (Kombination von 2-Acetacetoxyethylmethacrylat mit Hydroxyethylmethacrylat HEMA)

3.
a)

| | |
|---|---|
| 2-Acetacetoxyethylmethacrylat | 10 Gew.% |
| HEMA | 30 Gew.% |
| Ethanol/Wasser (1:1) | 60 Gew.% |
| Dentinhaftung (24h/H₂O/37°C): 18 ± 5 MPa (bei 1 von 6 Proben kohäsiver Bruch) Dentinhaftung (6 Monate/H₂O/37°C): 15 ± 3 MPa (bei 2 von 6 Proben kohäsive Brüche) | |

b)

| | |
|---|---|
| 2-Acetacetoxyethylmethacrylat | 20 Gew.% |
| HEMA | 20 Gew.% |
| Ethanol/Wasser (1:1) | 60 Gew.% |
| Dentinhaftung (24h/H₂O/37°C): 13 ± 6 MPa (bei 2 von 6 Proben kohäsive Brüche) | |

c)

| | |
|---|---|
| 2-Acetacetoxyethylmethacrylat | 40 Gew.% |
| HEMA | 20 Gew.% |
| Ethanol/Wasser (1:1) | 40 Gew.% |
| Dentinhaftung (24h/H₂O/37°C): 15 ± 6 MPa (bei 2 von 6 Proben kohäsive Brüche) | |

### Beispiel 4 (Kombination von MATP mit PEG-1000-DMA)

1.
a)

| | |
|---|---|
| MATP | 5 Gew.-% |
| PEG-1000-DMA | 30 Gew.-% |
| Ethanol/Wasser (1:1) | 65 Gew.-% |
| Dentinhaftung (24h/H₂O/37°C: 35 ± 6 MPa (alle Brüche kohäsiv) | |

b)

| | |
|---|---|
| MATP | 10 Gew.-% |
| PEG-1000-DMA | 30 Gew.-% |
| Ethanol/Waser (1:1) | 60 Gew.-% |
| Dentinhaftung (24h/H₂O/37°C): 17 ± 9 MPa (80 % der Brüche kohäsiv) | |

2.

| | |
|---|---|
| MATP | 10 Gew.-% |
| Glutaraldehyd (50 %-ig) | 30 Gew.-% |
| Ethanol/Wasser (1:1) | 60 Gew.-% |
| Dentinhaftung (24h/H₂O/37°C): 34 ± 8 MPa (alle Brüche kohäsiv) | |

3.

| | |
|---|---|
| MATP | 10 Gew.-% |
| HEMA | 30 Gew.-% |
| Ethanol/Wasser (1:1) | 60 Gew.-% |
| Dentinhaftung (24h/H₂O/37°C): 33 ± 11 MPa (alle Brüche kohäsiv) | |

### Beispiel 5 (Schmelzhaftung)

Der Schmelz wurde mit 1000-er Sandpapier angeschliffen.
1. Adhäsiv wurde direkt auf den Schmelz aufgetragen.

| | |
|---|---|
| MATP | 10 Gew.-% |
| HEMA | 30 Gew.-% |
| Ethanol/Wasser (1:1) | 60 Gew.-% |
| Schmelzhaftung (24h/H₂O/37°C): 3,7 ± 2,7 MPa | |

2. Syntac-Primer wurde zuerst auf den Schmelz aufgetragen.
a)

| | |
|---|---|
| MATP | 5 Gew.-% |
| PEG-1000-DMA | 30 Gew.-% |
| Ethanol/Wasser (1:1) | 65 Gew.-% |
| Schmelzhaftung (24h/H₂O/37°C): 22,3 ± 4,9 MPa | |

b)

| | |
|---|---|
| MATP | 10 Gew.-% |
| PEG-1000-DMA | 30 Gew.-% |
| Ethanol/Wasser (1:1) | 60 Gew.-% |
| Schmelzhaftung (24h/H₂O/37°C): 24,5 ± 5,3 MPa | |

c)

| | |
|---|---|
| MATP | 10 Gew.-% |
| Glutaraldehyd (50 %-ig) | 30 Gew.-% |
| Ethanol/Wasser (1:1) | 60 Gew.-% |
| Schmelzhaftung (24h/H₂O/37°C): 26,9 ± 10,7 MPa | |

d)

| | |
|---|---|
| MATP | 10 Gew.-% |
| HEMA | 30 Gew.-% |
| Ethanol/Wasser (1:1) | 60 Gew.-% |
| Schmelzhaftung (24h/H₂O/37°C): 22,2 ± 15,1 MPa | |

### Beispiel 6 (Metallhaftung)

Zunächst wurde die Haftung auf sandgestrahltem Wiron 88 (Ni/Cr/Mo-Legierung der Firma Bego) untersucht.
1.
a)

| | |
|---|---|
| Acetoacetoxyethyl-MMA | 20,0 Gew.% |
| Bis-GMA | 10,0 Gew.-% |
| Tetraethylenglykol-DMA | 20,0 Gew.-% |
| Aceton | 49,6 Gew.-% |
| Campherchinon | 0,15 Gew.-% |
| N-Cyanoethyl-N-methyl-anilin | 0,25 Gew.-% |

Mit dem Beispiel wird eine dünne Schicht dieser Lösung auf die Metalloberfläche aufgetragen und trocknen gelassen. Danach wird Opaquer-Pulver (Ivoclar) mit dieser Lösung angemischt und dünn aufgetragen. Nach dem Trocknen wird 60 Sek. mit dem Heliomat belichtet. Anschließend wird Helioprogress aufgetragen und 3 x 40 Sek. auspolymerisiert.
Metallhaftung (24h/H₂O/37°C): 14,9 ± 1,7 MPa
b)

| | |
|---|---|
| Acetoacetoxyethyl-MMA | 8,0 Gew.-% |
| Bis-GMA | 4,0 Gew.-% |
| Tetraethylenglykol-DMA | 8,0 Gew.-% |
| Aceton | 79,8 Gew.-% |
| Campherchinon | 0,07 Gew.-% |
| Cyanoethyl-methyl-anilin | 0,13 Gew.-% |

Vorgehensweise wie oben beschrieben.
Metallhaftung (24h/H₂O/27°C): 16.3 ± 1,8 MPa
Vergleichsweise wurde mit dem bestehenden Spektra-Link (Ivoclar) eine Metallhaftung von 16,1 ± 2,7 MPa erzielt.

Die angegebenen Werte für die Haftung sind als Relativwerte und nicht als Absolutwerte anzusehen, da bei einem Vergleich der Ergebnisse mehrerer Testreihen der identischen Experimente die Relationen zwar gleich sind, die ermittelten Werte aber im Vergleich der Testreihen untereinander voneinander abweichen können. Aufgrund der streuenden Qualität der verwendeten extrahierten Zähne werden in manchen Fällen bei etwas niedrigeren Haftwerten mehr kohäsive Brüche beobachtet.

Im Vergleich zu den bislang bekannten Dentalklebstoffen ergeben sich insgesamt gesehen erheblich verbesserte Haftwerte, was zu einer kleineren Anzahl adhäsiver Brüche und einer größeren Anzahl kohäsiver Brüche führt.

Bei Austausch des in den oben beschriebenen Formulierungen verwendeten erfindungsgemäßen Dentalklebstoffs durch einen der bekannten Klebstoffe "Syntac®, Gluma®" Scotchbond® 2, Tenure®" werden Dentinhaftwerte im Bereich von 5 bis 8 MPa gemessen.

### Beispiel 7 (Vergleichsbeispiel)

Bei diesem Beispiel wurde Scotchbond® 2 (von 3M) verwendet.
- Primer =: 2,5 % Maleinsäure 58,5 % Hydroxyethylmethacrylat (HEMA) in Wasser
- Klebstoff =: 62,5 % Bis-GMA 37,5 % Hydroxyethylmethacrylat (HEMA) Photoinitiatoren
(nach G.H. Johnson, V. Powell und G.E. Gordon; Dentin Bonding Systems: A Review of Current Products and Techniques; J. Am. Dent. Assoc. 122, 34 (1991))

Die Anwendung von Primer und Klebstoff erfolgte laut Herstellerangaben.

| | |
|---|---|
| Dentinhaftung (24h/H₂O/37°C): | 8 ± 2 MPa* |
| Dentinhaftung (1 Minute): | 2 ± 2 MPA* |

| | |
|---|---|
| * Es wurden nur adhäsive Brüche beobachtet. | |

### Beispiel 8 (Vergleichsbeispiel)

Bei diesem Beispiel wurde Gluma® (von Bayer) verwendet.
- Dentin-Reiniger =: 16 %-ige EDTA-Lösung
- Primer =: 35 % Hydroxyethylmethacrylat (HEMA) 5 % Glutaraldehyd in Wasser
- Versiegeler =: Bis-GMA
(nach G.H. Johnson et al.)

Die Anwendung von Dentin-Reiniger, Primer und Versiegeler erfolgte laut Herstellerangaben.

| | |
|---|---|
| Dentinhaftung (24h/H₂O/37°C): | 7 ± 3 MPa* |
| Dentinhaftung (1 Minute): | 3 ± 2 MPA* |

| | |
|---|---|
| * Es wurden nur adhäsive Brüche beobachtet. | |

## Patentansprüche

1. Verwendung einer polymerisierbaren mono- oder polyfunktionellen Carbonylverbindung, die a) mindestens eine polymerisierbare Vinylgruppe und b) mindestens eine Carbonylgruppe und in β-Position dazu eine zweite Carbonylgruppe oder eine andere funktionelle Gruppe aufweist, wobei die Carbonylverbindung einer der folgenden allgemeinen Formeln entspricht: 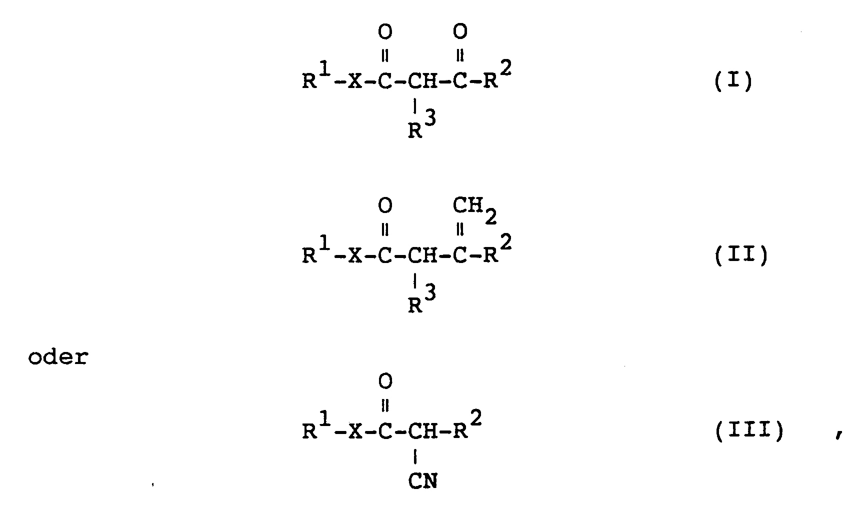 in denen
R¹ eine Alkyl-, Cycloalkyl-, Alkoxy- oder Arylgruppe oder eine Kombination daraus sein kann,
R² eine Alkyl-, Cycloalkyl-, Alkoxy- oder Arylgruppe, eine Kombinationen daraus oder Wasserstoff sein kann,
R³ eine Alkyl-, Cycloalkyl-, Alkoxy- oder Arylgruppe, eine Kombination daraus oder Wasserstoff sein kann, und
X entweder nicht vorhanden ist oder O, NR, NH oder S sein kann, wobei R eine Alkyl-, Cycloalkyl-, Alkoxy- oder Arylgruppe oder eine Kombination daraus sein kann, und
mindestens einer der Reste R¹, R² oder R³ außerdem durch einen die mindestens eine polymerisierbare Vinylgruppe enthaltenden Rest Y substituiert ist, ausgenommen Verbindungen, die mit Carbonsäureanhydridfunktionen substituierte Naphthylgruppen enthalten,
zum dauerhaften und festen Verbinden eines zu behandelnden Zahnmaterials mit einem Dentalwerkstoff.

2. Verwendung nach Anspruch 1, dadurch gekennzeichnet, daß Y ein unsubstituierter oder substituierter Acryl- oder Methacrylsäure-, Styryl-, Vinyl- oder Allylrest ist.

3. Verwendung nach Anspruch 1 oder 2, dadurch gekennzeichnet, daß Y durch Carboxylgruppen, die Teil einer Ester-, Amid- oder Thioestergruppe sein können, Hydroxygruppen, Alkylgruppen, Halogenatome und/oder Cyanidgruppen substituiert ist.

4. Verwendung nach einem der Ansprüche 1 bis 3, dadurch gekennzeichnet, daß die Carbonylverbindung eine der folgenden Formeln hat: 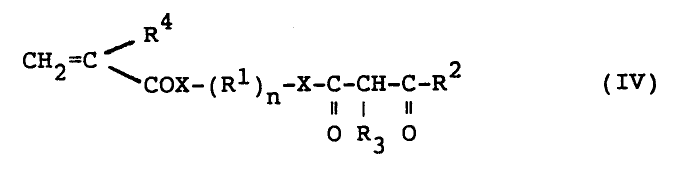
R¹ = Alkylen, Cycloalkylen, Alkylenoxy, Arylen,
R² = Alkyl, Alkoxy, Aryl, H,
R³ = Alkyl, Alkoxy, Aryl, H,
R⁴ = Alkyl, Hal, H, CN,
X = keine Bedeutung oder O, NR, NH, S, wobei R wie in Anspruch 1 definiert ist,
n = 1 und im Fall einer Alkylenoxygruppe bis zu 50,
wobei X jeweils gleich oder verschieden sein kann.

5. Verwendung nach einem der Ansprüche 1 bis 4, dadurch gekennzeichnet, daß die Carbonylverbindung ist:

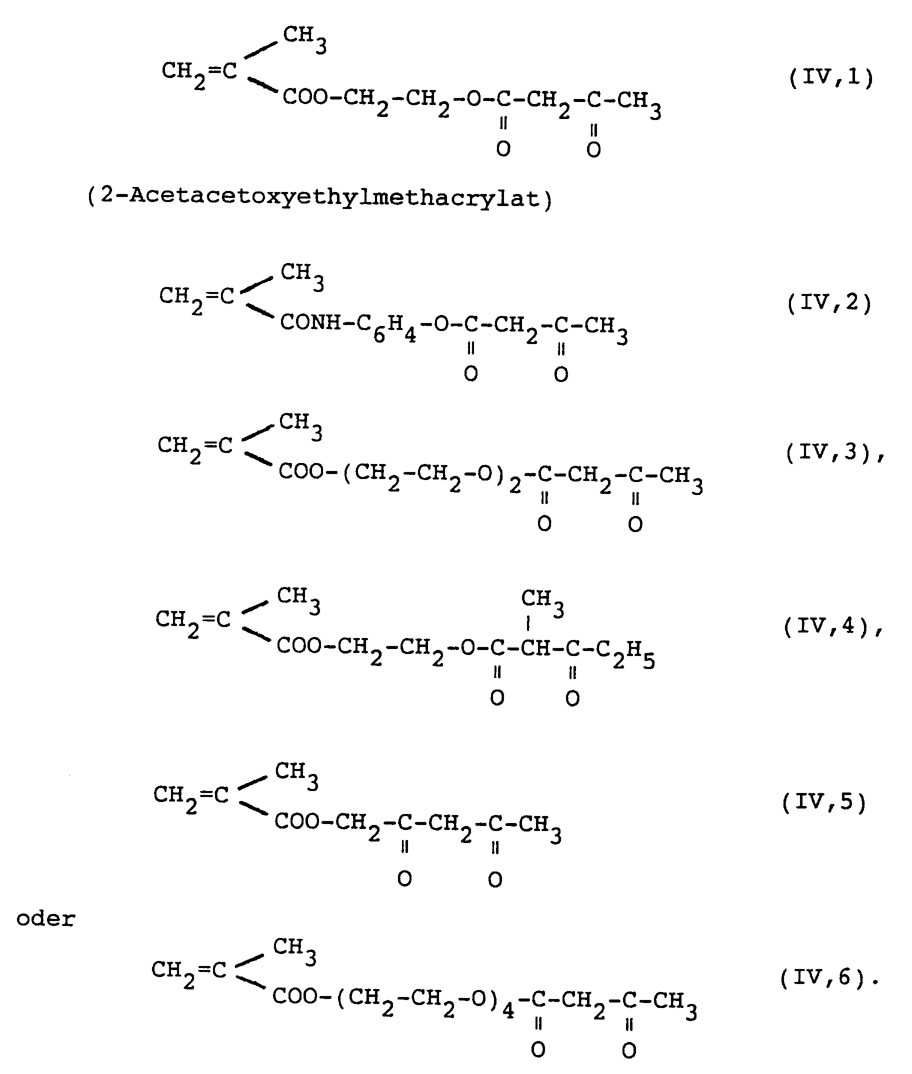

6. Verwendung nach Anspruch 1, dadurch gekennzeichnet, daß die Carbonylverbindung mehrere polymerisierbare Vinylgruppen und/oder mehrere Carbonylgruppen mit einer zweiten Carbonylgruppe oder einer anderen funktionelle Gruppe in β-Position dazu aufweist.

7. Verwendung nach einem der Ansprüche 1 bis 6, dadurch gekennzeichnet, daß die eingesetzte Zusammensetzung 1 bis 70, insbesondere 1 bis 50 und bevorzugt 5 bis 40 Gew.% der Carbonylverbindung enthält.

## Claims

1. Use of a polymerizable mono- or polyfunctional carbonyl compound which contains a) at least one polymerizable vinyl group and b) at last one carbonyl group and, in the β-position relative to this, a second carbonyl group or another functional group, the carbonyl compound corresponding to one of the following general formulae: 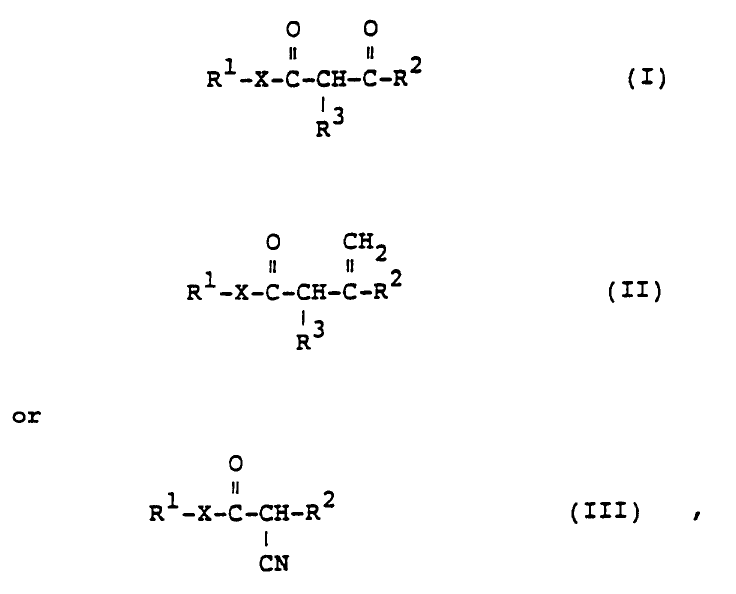 in which
R¹ can be an alkyl, cycloalkyl, alkoxy or aryl group or a combination thereof,
R² can be an alkyl, cycloalkyl, alkoxy or aryl group, a combination thereof or hydrogen,
R³ can be an alkyl, cycloalkyl, alkoxy or aryl group, a combination thereof or hydrogen and
X is either not present or can be O, NR, NH or S, where
R can be an alkyl, cycloalkyl, alkoxy or aryl group or a combination thereof, and
at least one of the radicals R¹, R² or R³ furthermore is substituted by a radical Y which contains at least one polymerizable vinyl group, excluding compounds which contain naphthyl groups substituted by carboxylic acid anhydride functions,
for permanent and firm bonding of a tooth material to be treated to a dental material.

2. Use according to Claim 1, characterized in that Y is an unsubstituted or substituted acrylic or methacrylic acid, styryl, vinyl or allyl radical.

3. Use according to Claim 1 or 2, characterized in that Y is substituted by carboxyl groups, which can be part of an ester, amide or thioester group, hydroxyl groups, alkyl groups, halogen atoms and/or cyanide groups.

4. Use according to one of Claims 1 to 3, characterized in that the carbonyl compound has one of the following formulae: 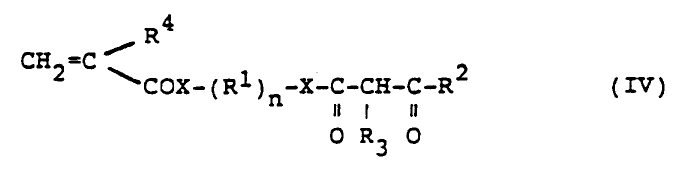
R¹ = alkylene, cycloalkylene, alkylenoxy or arylene,
R² = alkyl, alkoxy, aryl or H,
R³ = alkyl, alkoxy, aryl or H,
R⁴ = alkyl, Hal, H or CN,
X = no meaning or 0, NR, NH or S, where R is defined in Claim 1, n = 1 and, in the case of an alkylenoxy group, up to 50,
where X can in each case be identical or different.

5. Use according to one of Claims 1 to 4, characterized in that the carbonyl compound is:

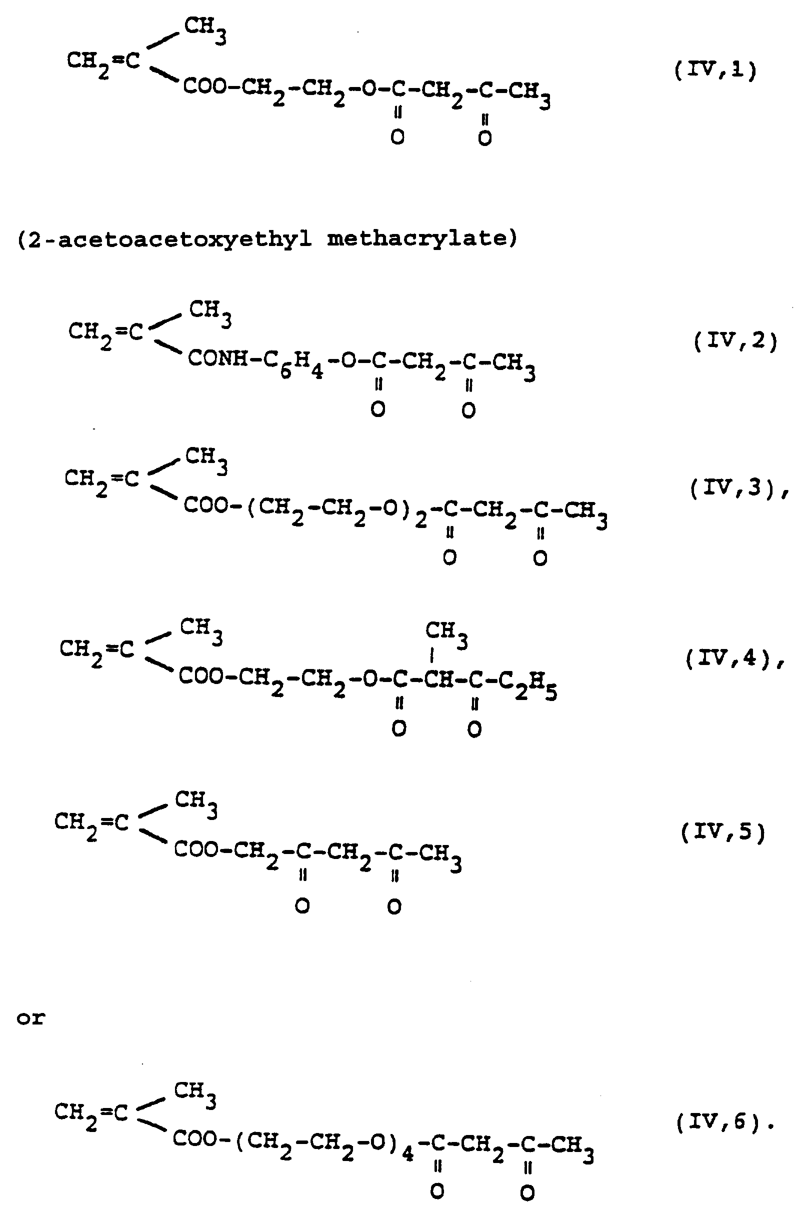

6. Use according to Claim 1, characterized in that the carbonyl compound contains several polymerizable vinyl groups and/or several carbonyl groups with a second carbonyl group or another functional group in the β-position relative to this.

7. Use according to one of Claims 1 to 6, characterized in that the composition employed comprises 1 to 70, in particular 1 to 50, and preferably 5 to 40% by weight of the carbonyl compound.

## Revendications

1. Utilisation d'un composé carbonyle polymérisable mono ou polyfonctionnel, qui présente a) au moins un groupe vinyle polymérisable et b) au moins un groupe carbonyle et un deuxième groupe carbonyle ou un autre groupe fonctionnel en position β par rapport au premier, dans laquelle le composé carbonyle correspond à l'une des formules générales suivantes : 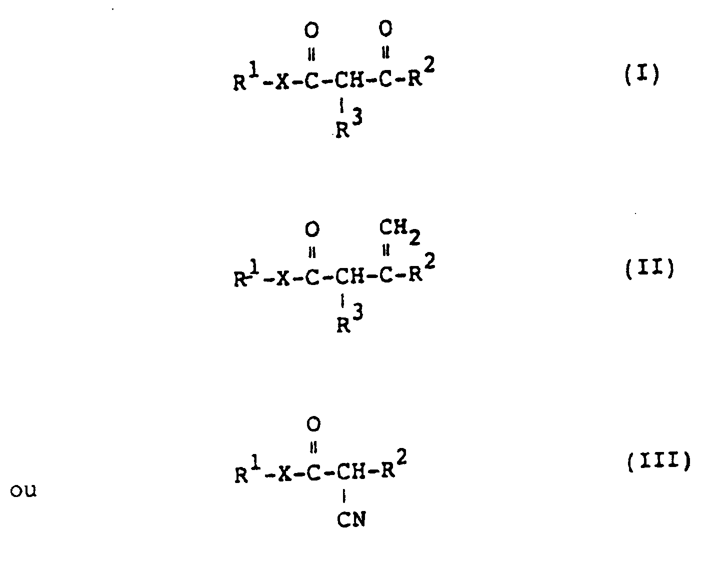 dans lesquelles
R¹ peut être un groupe alkyle, cycloalkyle, alcoxy, aryle ou une combinaison de ceux-ci,
R² peut être un groupe alkyle, cycloalkyle, alcoxy, aryle, une combinaison de ceux-ci ou un atome d'hydrogène,
R³ peut être un groupe alkyle, cycloalkyle, alcoxy, aryle, une combinaison de ceux-ci ou un atome d'hydrogène, et
X ou n'est pas présent ou peut être O, NR, NH ou S, R pouvant être un groupe alkyle, cycloalkyle, alcoxy ou aryle ou une combinaison de ceux-ci, et
formules dans lesquelles au mois un des radicaux R¹, R² ou encore R³ est substitué par un résidu Y contenant au moins un groupe vinyle polymérisable, à l'exception des composés qui contiennent des groupes naphtyles substitués par des fonctions anhydride d'acide carboxylique,
pour lier de manière durable et solide un matériau d'une dent à traiter avec un matériau dentaire.

2. Utilisation selon la revendication 1, caractérisée en ce que, Y est un radical d'acide acrylique ou méthacrylique, styryle, vinyle ou allyle substitué ou non substitué.

3. Utilisation selon la revendication 1 ou 2, caractérisée en ce que, Y est substitué par des groupes carboxyle, qui peuvent être une partie d'un groupe ester, amide ou thioester, des groupes hydroxy, des atomes d'halogène et/ou des groupes cyanure.

4. Utilisation selon l'une des revendications 1 à 3, caractérisée en ce que, le composé carbonyle a la formule suivante : 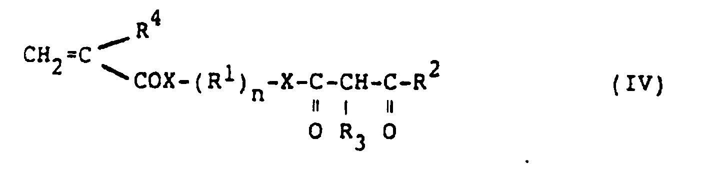
R¹ = alkylène, cycloalkylène, alkylènoxy, arylène,
R² = alkyle, alcoxy, aryle, H,
R³ = alkyle, alcoxy, aryle, H,
R⁴ = alkyle, un atome d'halogène, H, CN,
X = pas de signification ou O, NR, NH, S, R étant défini comme dans la revendication 1,
n = 1 et jusqu'à 50 dans le cas d'un groupe alkylènoxy,
caractérisé en ce que chaque X peut être identique ou différent.

5. Utilisation selon l'une des revendications 1 à 4, caractérisée en ce que, le composé carbonyle est :

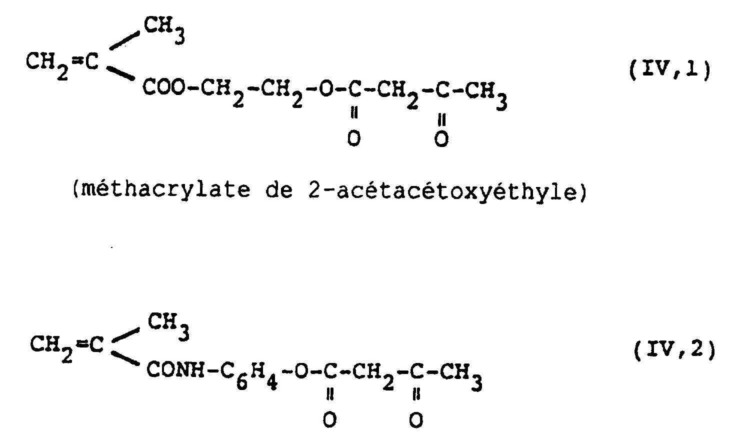

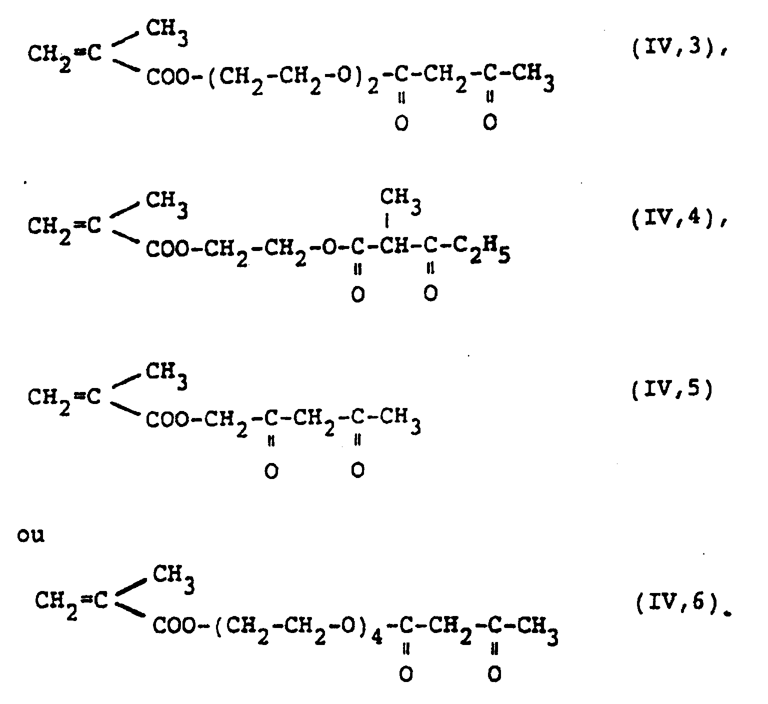

6. Utilisation selon la revendication 1, caractérisée en ce que, le composé carbonyle présente plusieurs groupes vinyle polymérisables et/ou plusieurs groupes carbonyle avec un deuxième groupe carbonyle ou un autre groupe fonctionnel en position β par rapport au premier.

7. Utilisation selon l'une des revendications 1 à 6, caractérisée en ce que la composition employée contient de 1 à 70, en particulier de 1 à 50 et de préférence de 5 à 40% en poids du composé carbonyle.
